# EUROPEAN PATENT APPLICATION

(11) **EP 0 983 760 A1**
(43) Date of publication of application: **08.03.2000**
(21) Application number: 98116643.2
(22) Date of filing: 03.09.1998
(51) Int. Cl.: A61F 13/58, A61F 13/62

(54) **Diaper fastening system**

(71) Applicant: THE PROCTER & GAMBLE COMPANY, Cincinnati, Ohio 45202 (US)
(72) Inventor: Tilly, Edgar, 65760 Eschborn (DE)
(74) Representative: Canonici, Jean-Jacques

(57) **Abstract**

A disposable diaper having an adhesive tab fastener in which a first securing tape ribbon has a free end and a fixed end secured to the diaper. A second securing tape ribbon is coextensive with and adhesively but releasably attached to the adhesive-coated face of the free working end of the first securing tape ribbon. The second tape ribbon includes a pressure-sensitive adhesive coating on a face thereof. A release means is provided on the diaper topsheet. The free end of the first securing tape ribbon is adapted to secure the diaper about an infant by adhesive attachment via the adhesive-coated face of the second securing tape ribbon. The free end of the first securing tape ribbon is separable from the second securing tape ribbon to permit the opening of an applied diaper for inspection, and to make the adhesive coating on the free end of the first securing tape ribbon available for use in refastening the diaper. A grippable portion of the free working end of said first tape ribbon projects beyond the respective outermost edges of the release means and the second securing tape ribbon.

## Description

### FIELD OF THE INVENTION

The present invention relates to articles which absorb and/or contain bodily exudates, including disposable absorbent articles such as diapers, adult incontinence products, sanitary napkins and the like. More particularly, the invention relates to disposable absorbent articles adapted to be secured in place by adhesive tabs.

### BACKGROUND OF THE INVENTION

Disposable diapers provide substantial advantages in convenience over diapers intended to be laundered and reused, particularly when they are used away from home. In recent years, many different disposable diapers have been proposed and some have been successful in the marketplace.

As may be seen from prior disposable diapers, it is desirable to obviate the problems that are inherent in closure systems which utilize extraneous fasteners such as safety pins, snaps and zippers. To this end adhesive closure systems have presented acceptable solutions which eliminate the need for pins, for example, which present problems especially when the infant is active during the diaper changing.

US Pat. No. 4,049,001 issued to Tritsch, discloses a diaper with a resealable tape closure. The tape closure includes a first securing tape ribbon and a second securing tape ribbon which is releasably attached to the first securing tape ribbon. One face of the second securing tape ribbon is provided with an adhesive coating. A portion of the first tape ribbon is adapted to secure the diaper about an infant by adhesive attachment via the adhesive-coated face of the second tape ribbon. A free end of the first tape ribbon is separable from the second tape ribbon to permit the opening of an applied diaper for inspection, and to make the adhesive coating on the first securing tape ribbon available for use in refastening the diaper about the infant.

A problem with the tape closure of Tritsch is that the first tape ribbon is difficult to grab as the distal portions of both the second tape ribbon and the release means extend beyond the distal portion of the first tape ribbon.

### SUMMARY OF THE INVENTION

The present invention is directed to an improved economical tape tab system which is particularly well suited for use in disposable diapers which permits an originally fastened diaper to be opened and reclosed several times without tearing the tape tab and without rupturing the diaper. Thus, the diaper can be opened and closed for inspection or adjustment many times during the normal service of the diaper. The original closure and the subsequent closings around the infant provide a good, strong adhesive attachment of the diaper.

The disposable diaper comprises a topsheet, a moisture-impervious backsheet substantially coextensive with said topsheet, an absorbent core positioned between said topsheet and said backsheet, and an adhesive tab fastener.

The adhesive tab fastener comprises a first securing tape ribbon having a fixed end and a free working end. An adhesive coating on at least one face of said fixed end of said first securing tape ribbon attaches the fixed end of said first securing tape ribbon to said disposable absorbent article. A pressure-sensitive adhesive coating is provided on one face of said free working end of said first securing tape ribbon.

A second securing tape ribbon is coextensive with and adhesively but releasably attached to said adhesive-coated face of said free working end of said first securing tape ribbon. The second tape ribbon includes a pressure-sensitive adhesive coating on said second securing ribbon on the face thereof opposite to the face attached to said free working end of said first securing tape ribbon.

A release means is provided on said topsheet. The second securing tape ribbon is movable from a folded-over storage position in which said second securing tape ribbon is releasably adhered to said release means to a working position in which said adhesive-coated second securing tape ribbon is available for use in securing said disposable absorbent article about an infant.

The free working end of said first securing tape ribbon is separable from said second securing tape ribbon to enable said disposable absorbent article to be removed from an infant and to make said pressure-sensitive adhesive coating on said free working end of said first securing tape ribbon available for use in refastening the disposable absorbent article about an infant.

A grippable portion of said free working end of said first tape ribbon projects beyond the respective outermost edges of said release means and said second securing tape ribbon.

The improved tape tab system of the present invention will allow someone inspecting or adjusting the diaper to do so many times during the service of the diaper. Upon refastening, the tape tabs provide a good, strong securement to the diaper.

### BRIEF DESCRIPTION OF THE DRAWINGS

While the specification concludes with claims particularly pointing out and distinctly claiming the subject matter which is regarded as the present invention, it is believed that the description will be better understood from the following descriptions which are taken in conjunction with the accompanying drawings in which like designations are used to designate substantially identical elements.
Figure 1 is a plan view of an absorbent article embodiment of the present invention having portions cut away to reveal the underlying structure, the garment-facing surface of the diaper facing the viewer.
Figure 2 is an enlarged fragmentary cross-sectional view of the diaper of Figure 1 taken along section line 2-2 of Figure 1;
Figure 3 is an enlarged fragmentary cross-sectional view of the diaper of Figure 1 taken along section line 3―3 of Figure 1, and showing in phantom the position which can be assumed by the detachable portion of the tab fastener of the present invention.
Figure 4 is an enlarged fragmentary cross-sectional view another embodiment of a fastener of the present invention.

### DETAILED DESCRIPTION OF THE INVENTION

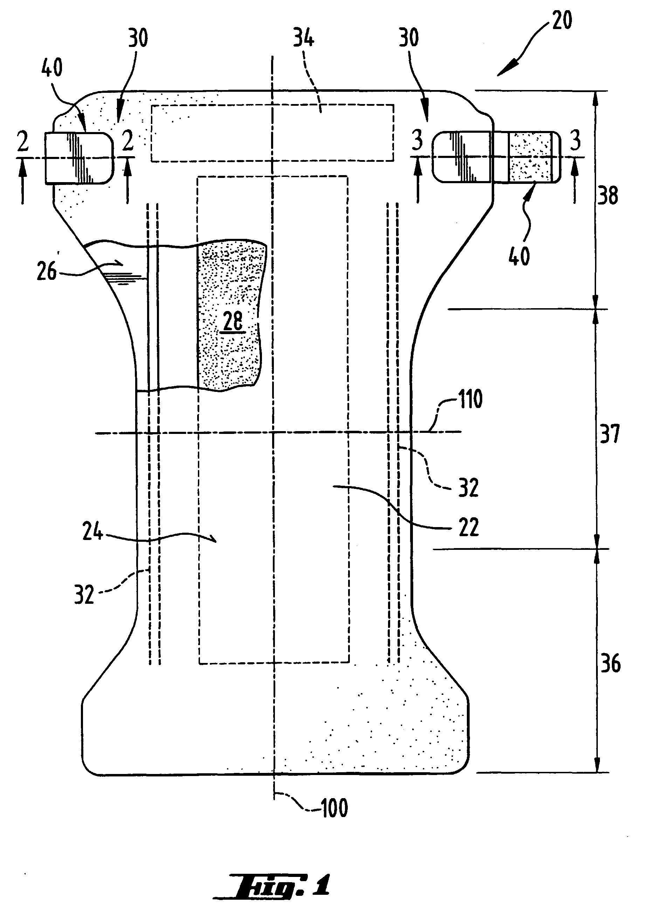

As used herein, the term "absorbent article" refers to devices which absorb and contain body exudates, and more specifically, refers to devices which are placed against or in proximity to the body of the wearer to absorb and contain the various exudates discharged from the body. The term "disposable" is used herein to describe absorbent articles which generally are not intended to be laundered or otherwise restored or reused as an absorbent article (i.e., they are intended to be discarded after a single use and, preferably, to be recycled, composted or otherwise disposed of in an environmentally compatible manner). (As used herein, the term "disposed" is used to mean that an element(s) of the diaper is formed (joined and positioned) in a particular place or position as a unitary structure with other elements of the diaper or as a separate element joined to another element of the diaper. As used herein, the term "joined" encompasses configurations whereby an element is directly secured to another element by affixing the element directly to the other element, and configurations whereby an element is indirectly secured to another element by affixing the element to intermediate member(s) which in turn are affixed to the other element.) A "unitary" absorbent article refers to absorbent articles which are formed of separate parts united together to form a coordinated entity so that they do not require separate manipulative parts like a separate holder and liner. A preferred embodiment of an absorbent article of the present invention is the unitary disposable absorbent article, diaper 20, shown in Figure 1. As used herein, the term "diaper" refers to an absorbent article generally worn by infants and incontinent persons about the lower torso. The present invention is also applicable to other absorbent articles such as absorbent bandages, incontinence briefs, incontinence undergarments, absorbent inserts, diaper holders and liners, feminine hygiene garments, and the like.

Figure 1 is a plan view of the diaper 20 of the present invention in a flat-out, state with portions of the structure being cut-away to more clearly show the construction of the diaper 20. The portion of the diaper 20 which faces the wearer is oriented towards the viewer. As shown in Figure 1, the diaper 20 preferably comprises a liquid pervious topsheet 24; a liquid impervious backsheet 26; an absorbent core 28, which is preferably positioned between at least a portion of the topsheet 24 and the backsheet 26; side panels 30; elasticized leg cuffs 32; an elastic waist feature 34; and fastening tabs 40. Diaper 20 is shown in Figure 1 to have a first waist region 36, a second waist region 38 opposed to the first waist region 36 and a crotch region 37 located between the first waist region and the second waist region. The periphery of the diaper 20 is defined by the outer edges of the diaper 20 in which the longitudinal edges run generally parallel to the longitudinal centerline 100 of the diaper 20 and the end edges run between the longitudinal edges generally parallel to the lateral centerline 110 of the diaper 20.

The chassis 22 of the diaper 20 comprises the main body of the diaper 20. The chassis 22 comprises at least a portion of the absorbent core 28 and preferably an outer covering layer including the topsheet 24 and the backsheet 26. If the absorbent article comprises a separate holder and a liner, the chassis 22 generally comprises the holder and the liner. (For example, the holder may comprise one or more layers of material to form the outer cover of the article and the liner may comprise an absorbent assembly including a topsheet, a backsheet, and an absorbent core. In such cases, the holder and/or the liner may include a fastening element which is used to hold the liner in place throughout the time of use.) For unitary absorbent articles, the chassis 22 comprises the main structure of the diaper with other features added to form the composite diaper structure. While the topsheet 24, the backsheet 26, and the chassis 22 may be assembled in a variety of well known configurations, preferred diaper configurations are described generally in U.S. Patent 3,860,003; U.S. Pat. No. 5,151,092; and U.S. Pat. No. 5,221. Other suitable diaper chassis design are disclosed in U.S. Pat. No. 5,569,232; U.S. Pat. No. 5,554,144; U.S. Pat. No. 5,554,143; U.S. Pat. No. 5,554,145; and U.S. Pat. No. 5,556,394. Each of these references is hereby incorporated by reference herein.

The backsheet 26 is generally that portion of the diaper 20 positioned adjacent the garment facing surface of the absorbent core 28 which prevents the exudates absorbed and contained therein from soiling articles which may contact the diaper 20, such as bedsheets and undergarments. In preferred embodiments, the backsheet 26 is impervious to liquids (e.g., urine) and comprises a thin plastic film such as a thermoplastic film having a thickness of about 0.012 mm (0.5 mil) to about 0.051 mm (2.0 mils). Suitable backsheet films include those manufactured by Tredegar Industries Inc. of Terre Haute, IN and sold under the trade names X15306, X10962 and X10964. Other suitable backsheet materials may include breathable materials which permit vapors to escape from the diaper 20 while still preventing exudates from passing though the backsheet 26. Exemplary breathable materials may include materials such as woven webs, nonwoven webs, composite materials such as film-coated nonwoven webs, and microporous films such as manufactured by Mitsui Toatsu Co., of Japan under the designation ESPOIR NO and by EXXON Chemical Co., of Bay City, TX, under the designation EXXAIRE. Suitable breathable composite materials comprising polymer blends are available from Clopay Corporation, Cincinnati, OH under the name HYTREL blend P18-3097. Such breathable composite materials are described in greater detail in PCT Application No. WO 95/16746, published on June 22, 1995 in the name of E. I. DuPont. Other breathable backsheets including nonwoven webs and apertured formed films are described in U.S. Pat. No. 5,571,096. Each of these references is hereby incorporated by reference herein.

The backsheet 26, or any portion thereof, may be elastically extensible in one or more directions. In one embodiment, the backsheet 26 may comprise a structural elastic-like film ("SELF") web. A structural elastic-like film web is an extensible material that exhibits an elastic-like behavior in the direction of elongation without the use of added elastic materials. The SELF web includes a strainable network having at least two contiguous, distinct, and dissimilar regions. Preferably, one of the regions is configured so that it will exhibit resistive forces in response to an applied axial elongation in a direction parallel to the predetermined axis before a substantial portion of the other region develops significant resistive forces to the applied elongation. At least one of the regions has a surface-pathlength which is greater than that of the other region as measured substantially parallel to the predetermined axis while the material is in an untensioned condition. The region exhibiting the longer surface-pathlength includes one or more deformations which extend beyond the plane of the other region. The SELF web exhibits at least two significantly different stages of controlled resistive force to elongation along at least one predetermined axis when subjected to an applied elongation in a direction parallel to the predetermined axis. The SELF web exhibits first resistive forces to the applied elongation until the elongation of the web is sufficient to cause a substantial portion of the region having the longer surface-pathlength to enter the plane of applied elongation, whereupon the SELF web exhibits second resistive forces to further elongation. The total resistive forces to elongation are higher than the first resistive forces to elongation provided by the first region. SELF webs suitable for the present invention are more completely described in U.S. Patent No. 5,518,801, which is incorporated herein by reference. In alternate embodiments, the backsheet 26 may comprise elastomeric films, foams, strands, or combinations of these or other suitable materials with nonwovens or synthetic films.

The backsheet 26 may be joined to the topsheet 24, the absorbent core 28 or any other element of the diaper 20 by any attachment means known in the art. For example, the attachment means may include a uniform continuous layer of adhesive, a patterned layer of adhesive, or an array of separate lines, spirals, or spots of adhesive. One preferred attachment means comprises an open pattern network of filaments of adhesive as disclosed in U.S. Patent 4,573,986. Other suitable attachment means include several lines of adhesive filaments which are swirled into a spiral pattern, as is illustrated by the apparatus and methods shown in U.S. Patent 3,911,173; U.S. Patent 4,785,996; and U.S. Patent 4,842,. Each of these patents are incorporated herein by reference. Adhesives which have been found to be satisfactory are manufactured by H. B. Fuller Company of St. Paul, Minnesota and marketed as HL-1258. Alternatively, the attachment means may comprise heat bonds, pressure bonds, ultrasonic bonds, dynamic mechanical bonds, or any other suitable attachment means or combinations of these attachment means as are known in the art.

The topsheet 24 is preferably positioned adjacent the body surface of the absorbent core 28 and may be joined thereto and/or to the backsheet 26 by any attachment means known in the art. Suitable attachment means are described above with respect to means for joining the backsheet 26 to other elements of the diaper 20. In one preferred embodiment of the present invention, the topsheet 24 and the backsheet 26 are joined directly to each other in some locations and are indirectly joined together in other locations by directly joining them to other elements of the diaper 20.

The topsheet 24 is preferably compliant, soft feeling, and non-irritating to the wearer's skin. Further, at least a portion of the topsheet 24 is liquid pervious, permitting liquids to readily penetrate through its thickness. A suitable topsheet 24 may be manufactured from a wide range of materials, such as porous foams; reticulated foams; apertured plastic films; or woven or nonwoven webs of natural fibers (e.g., wood or cotton fibers), synthetic fibers (e.g., polyester or polypropylene fibers), or a combination of natural and synthetic fibers. If the absorbent assemblies include fibers, the fibers may be spunbond, carded, wet-laid, meltblown, hydroentangled, or otherwise processed as is known in the art. One suitable topsheet 24 comprising a web of staple length polypropylene fibers is manufactured by Veratec, Inc., a Division of International Paper Company, of Walpole, Massachusetts under the designation P-8.

Preferably, the topsheet 24 is made of a hydrophobic material or is treated to be hydrophobic in order to isolate the wearer's skin from liquids contained in the absorbent core 28. If the topsheet 24 is made of a hydrophobic material, preferably at least the upper surface of the topsheet 24 is treated to be hydrophilic so that liquids will transfer through the topsheet more rapidly. This diminishes the likelihood that body exudates will flow off the topsheet 24 rather than being drawn through the topsheet 24 and being absorbed by the absorbent core 28. The topsheet 24 can be rendered hydrophilic by treating it with a surfactant or by incorporating a surfactant into the topsheet. Suitable methods for treating the topsheet 24 with a surfactant include spraying the topsheet 24 material with the surfactant and immersing the material into the surfactant. A more detailed discussion of such a treatment and hydrophilicity is contained in U.S. Pat. No. 4,988,344 and U.S. Pat. No. 4,988,345. A more detailed discussion of some suitable methods for incorporating surfactant in the topsheet can be found in U.S. Statutory Invention Registration No. H1670, published on July 1, 1997 in the names of Aziz et al. Each of these references is hereby incorporated by reference herein.

Any portion of the topsheet 24 may be coated with a lotion as is known in the art. Examples of suitable lotions include those described in U.S. Pat. No. 5,607,760; U.S. Pat. No. 5,609,587; U.S. Pat. No. 5,635,191; and U.S. Pat. No. 5,643,588. The topsheet may also include or be treated with antibacterial agents, some examples of which are disclosed in PCT Publication No. WO 95/24173 entitled "Absorbent Articles Containing Antibacterial Agents in the Topsheet For Odor Control" which was published on September 14, 1995 in the name of Johnson. Further, the topsheet 24, the backsheet 26 or any portion of the topsheet or backsheet may be embossed and/or matte finished to provide a more cloth like appearance.

The absorbent core 28 may comprise any absorbent material which is generally compressible, conformable, non-irritating to the wearer's skin, and capable of absorbing and retaining liquids such as urine and other certain body exudates. The absorbent core 28 may be manufactured in a wide variety of sizes and shapes (e.g., rectangular, hourglass, "T"-shaped, asymmetric, etc.) and may comprise a wide variety of liquid-absorbent materials commonly used in disposable diapers and other absorbent articles such as comminuted wood pulp, which is generally referred to as airfelt. Examples of other suitable absorbent materials include creped cellulose wadding; meltblown polymers, including coform; chemically stiffened, modified or cross-linked cellulosic fibers; tissue, including tissue wraps and tissue laminates; absorbent foams; absorbent sponges; superabsorbent polymers; absorbent gelling materials; or any other known absorbent material or combinations of materials.

The configuration and construction of the absorbent core 28 may also be varied (e.g., the absorbent core(s) or other absorbent structure(s) may have varying caliper zones, a hydrophilic gradient, a superabsorbent gradient, or lower average density and lower average basis weight acquisition zones; or may comprise one or more layers or structures). However, the total absorbent capacity of the absorbent core 28 should be compatible with the design loading and the intended use of the diaper 20.

Exemplary absorbent structures for use as the absorbent assemblies are described in U.S. Patent 4,610,678; U.S. Patent 4,673,402; U.S. Patent 4,834,735; U.S. Patent 4,888,231; U.S. Pat. No. 5,137,537; U.S. Patent 5,147,345; and U.S. Pat. No. 5,342,338. Each of these patents is incorporated herein by reference.

The diaper 20 may also comprise at least one elastic waist feature 34 that helps to provide improved fit and containment. The elastic waist feature 34 is generally intended to elastically expand and contract to dynamically fit the wearer's waist. The elastic waist feature 34 preferably extends at least longitudinally outwardly from at least one waist edge 62 of the absorbent core 28 and generally forms at least a portion of the end edge 52 of the diaper 20. Disposable diapers are often constructed so as to have two elastic waist features, one positioned in the first waist region 36 and one positioned in the second waist region 38. Further, while the elastic waist feature 34 or any of its constituent elements may comprise one or more separate elements affixed to the diaper 20, the elastic waist feature 34 may be constructed as an extension of other elements of the diaper 20, such as the backsheet 26, the topsheet 24, or both the backsheet 26 and the topsheet 24.

The elastic waist feature 34 may be constructed in a number of different configurations including those described in U.S. Patent 4,515,595; U.S. Patent 4,710,189; U.S. Pat. No 5, 151,092; and U.S. Pat. No. 5,221,274. Other suitable waist configurations may include waistcap features such as those described in U.S. Patent 5,026,364 and U.S. Patent 4,816,025. All of the above mentioned references are incorporated herein by reference.

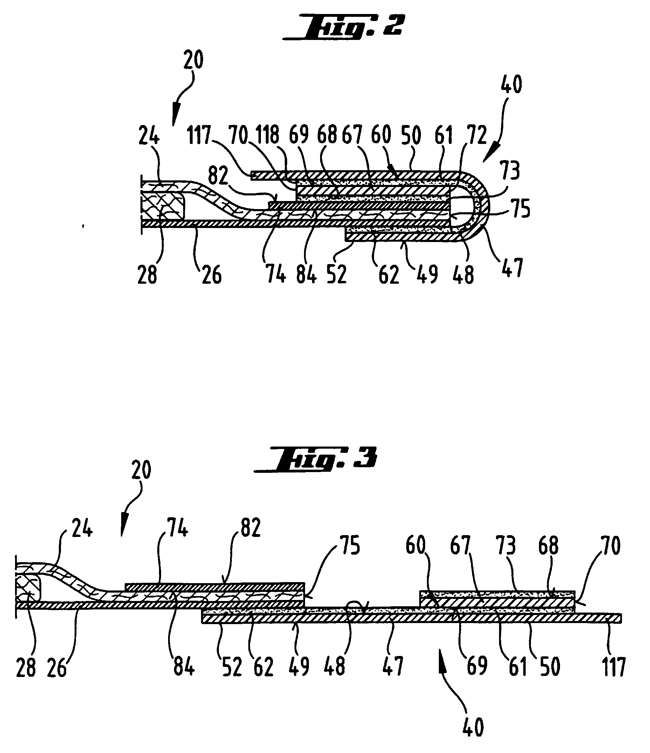

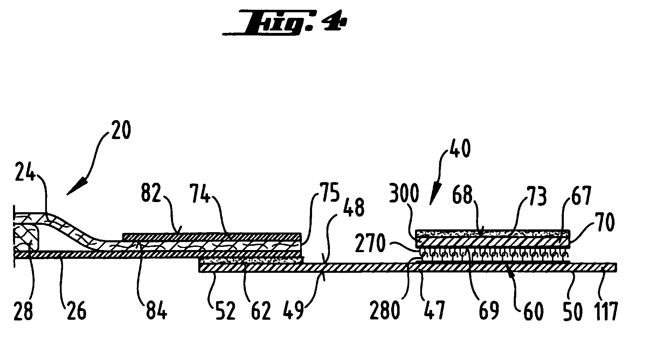

Fasteners such as tabs 40 are attached to diaper 20 for securing the diaper about a wearer. As described in greater detail below, tabs 40 are movable from a folded-over storage position illustrated in Figure 2 to a working position which is illustrated in Figure 3. As illustrated in Figures 2 and 3, tab 40 includes a first securing tape ribbon 47 having an inner face 48 and a outer face 49. First securing tape ribbon 47 has a free working end 50 and a fixed end 52 which is permanently attached to a marginal portion of diaper 20, preferably along backsheet 26.

Securement means such as adhesive coatings are provided on a least one face of fixed end 52. Securement means on both faces of fixed end 52 is desirable when the fixed end 52 is sandwiched between topsheet 24 and backsheet 26 to distribute forces therebetween. As shown in Figures 2 and 3, fixed end 52 is provided with adhesive coating 62 on inner face 48 thereof. Fixed end 52 of first securing tape ribbon 47 is attached to backsheet 26 by means of adhesive coating 62 which can be made of a pressure-sensitive adhesive composition, a heat-activated or solvent-activated adhesive composition, or the like.

Securement means 60 is provided on one face of free working end 50. As shown in Figures 2 and 3, securement means 60 on free working end 50 comprises an adhesive coating 61. Adhesive coating 61 is preferably pressure-sensitive and coextensive with free working end 50 but for a grippable distal end portion 117.

Tab 40 further includes second securing tape ribbon 67 having opposing inner and outer faces 68 and 69. Outer face 69 of second securing tape ribbon 67 is substantially coextensive with, and releasably attached to, the first face 48 of free working end 50 of first securing tape ribbon 47. The attachment or bond between second tape ribbon 67 and free working end 50 is at least as strong as the attachment or bond between fixed end 52 and backsheet 26 so that ribbons 47 and 67 effectively perform as a single tab or ribbon during the initial diaper securement. Grippable distal end portion 117 of first securing tape ribbon 47 extends beyond first end edge 70 of second securing tape ribbon 67. A continuous adhesive coating 73 is provided along inner face 68 of the entire second securing tape ribbon 67. Adhesive coating 73 is preferably a pressure-sensitive adhesive composition.

Release means 74 is adapted to be releasably attached to adhesive coating 73 on second securing tape ribbon 67. In the embodiment illustrated in Figures 2 and 3, release means 74 is carried by diaper 20 at a marginal location thereon to provide a release region facing in the same direction as topsheet 24.

Second securing tape ribbon 67 is releasable from release means 74 to make pressure-sensitive adhesive coating 73 available for use in securing diaper 20 about an infant. Referring to Figures 2 and 3, first securing tape ribbon 47 and second securing tape ribbon 67 are folded about longitudinal edge 75 of diaper 20. Second securing tape ribbon 67 co-acts with first securing tape ribbon 67 and provides securement means for initially fastening diaper 20 about an infant. The securement means can be moved from the folded-over, storage position of Figure 2 in which adhesive coating 73 is releasably adhered to release means 74, to the extended, working position of Figure 3 in which the adhesive coating 73 on inner face 68 of second securing tape ribbon 67 is available for use in securing diaper 20 about an infant. Adhesive coating 73 faces in the same direction as diaper topsheet 24 when second securing tape ribbon 67 is in the working position.

Thus, when tab 40 is in the extended working position of Figure 3, free working end 50 of first securing tape ribbon 47 is adapted to secure diaper 20 about an infant by adhesive attachment to the outside surface or backsheet 26 of the diaper via the second securing tape ribbon 67 which is carried entirely by free working end 50. Free working end 50 of first securing tape ribbon 47 is separable from second securing tape ribbon 67 to enable diaper 20 to be opened or removed from the infant, and to make adhesive coating 61 on free working end 50 of first securing tape ribbon 47 available for use in refastening diaper 20 about an infant. Free working end 50 of first securing tape ribbon 47 is thereby made detachable from and refastenable to second securing tape ribbon 67 for inspecting and/or repositioning the diaper about an infant. Free working end 50 is movable from an extended working position wherein free working end 50 is adhesively attached to second securing tape ribbon 67, to a detached position wherein free working end 50 of first securing tape ribbon 47 is separated from second securing tape ribbon 67 which remains fastened to the diaper.

### HERE

Diaper 20 can be fastened with tab 40 as depicted in Figure 3. Fixed end 52 of first securing tape ribbon 47 is adhesively attached to one corner of diaper 20. When tab 40 is unfolded from the storage position of Figure 2 to the working position of Figure 3, tab 40 is adhesively fastened to an opposite corner of diaper 20 by means of adhesive coating 73 on second securing tape ribbon 67. The fastening of first securing tape ribbon 47 to diaper 20 via second securing tape ribbon 67 provides a good, strong closure means around the infant. When the diaper is in the fastened condition, second securing tape ribbon 67 lies between first securing tape ribbon 47 and backsheet 26 or the diaper's outside surface.

It is a feature of the present invention that when the diaper needs inspection or adjustment after an original closure has been made, the first securing tape ribbon 47 can be peeled from second securing tape ribbon 67 without disturbing the adhesive attachment of second securing tape ribbon 67 to backsheet 26. This can be accomplished because the first securing tape ribbon 47 is adhesively but releasably attached to second securing tape ribbon 67 and not to any portion of backsheet 26. The grippable end 117 of first securing tape ribbon 47 extends beyond the end edge 70 of second securing tape ribbon 67, to facilitate separation of first securing tape ribbon 47 from second securing tape ribbon 67.

First securing tape ribbon 47 can therefore be separated from second securing tape ribbon 67 to open a fastened diaper without resorting to tearing the backsheet 26 or the tab itself When first securing tape ribbon 47 is separated from second securing tape ribbon 67, fixed end 52 of first securing tape ribbon 47 remains secured to one corner of diaper 20 while the second securing tape ribbon 67 remains fastened to the opposite corner of diaper 20 where the original closure was made. Second securing tape ribbon 67 acts as a reinforcing agent by remaining on the diaper where the original closure was made. The second securing tape ribbon adds strength to the area of the diaper which might otherwise tear upon peeling of first securing tape ribbon 47 from second securing tape ribbon 67 due to the stresses imposed on diaper 20 by the peeling action. When first and second tape ribbons 47 and 67 are separated from one another, diaper 20 can be inspected for soiling and/or can be readjusted for a better or neater fit around the infant.

When the inspection and/or adjustment is completed, diaper 20 is wrapped around the infant as was done originally and is refastened by positioning first securing tape ribbon 47 in an overlapping relationship with second securing tape ribbon 67 which remains attached to the opposite corner of diaper 20. Adhesive coating 61 on free working end 50 of first securing tape ribbon 47 is pressed against outer face 69 of second securing tape ribbon 67 to complete the closure. Since free working end 50 of first securing tape ribbon 47 is longer than second securing tape ribbon 67 the grippable end 117 is easy to grasp making additional detachments and refastenings of the diaper convenient for the care provider. The refastened diaper is provided with a strong adhesive attachment because the adhesive material comprising adhesive coating 61 on free working end 50 remains thereon after the original closure is broken. The strength of the adhesive permits many openings and closures of the diaper.

As shown in Figures 2 and 3, release means 74 may comprise a ribbon segment or release strip having a release-coated surface on face 82 which provides the release region, and an adhesive coating on opposite face 84 by means of which the release strip is anchored to topsheet 24. Alternatively, the release means may comprise a release layer which is a surface coating on a marginal position of the topsheet 24, and preferably comprises a silicone release compound, or the like. The release strip or release layer preferably provides a release region of about the same width as tab 40 and is substantially coextensive with adhesive coating 73 on second securing tape ribbon 67. However, the release region may have a greater width than second securing tape ribbon 67 so as to provide for manufacturing tolerances.

While the distance between the end of securing tape ribbon 67 and diaper longitudinal edge or margin 75 is not overly critical, this distance preferably should be sufficiently small so as to preclude substantial adhesion of a juxtaposed region of backsheet 26 to an exposed adhesive region of pressure sensitive adhesive carried on face 48 when the diaper is releasably secured about an infant by means of securing tape ribbon 67. Undesirable adhesion can also be avoided by positioning tab 40 on diaper 20 so that the innermost edge of fixed end 72 abuts longitudinal edge 75. The exposed adhesive region on inner face 48 can also be covered, if necessary or desirable, with a sheet or film material such as polyethylene, polyethylene terephthalate, cellulose, paper, or the like. In the alternative, an adhesive-free zone can be provided on the exposed region of face 48 between adhesive coatings 61 and 62. Yet another possibility is to position release strip 74 so that the outermost end thereof extends beyond diaper edge 75 and a projecting portion of release strip 74 covers the exposed adhesive mass.

A gripping means 117 is provided to facilitate separation of release means 74 and second securing tape ribbon 67 to expose adhesive coating 73 of second securing tape ribbon 67 preparatory to fastening the diaper about an infant. When the user pulls gripping means 117 of first securing tape ribbon 47, second securing tape ribbon 67 separates from release means 74 to expose adhesive coating 73 of second securing tape ribbon 67 as the adhesive forces between first securing tape ribbon 47 and second securing tape ribbon 67 are stronger than the adhesive forces between second securing tape ribbon 67 and release means 74.

Gripping means 117 also facilitates in the separation of first securing tape ribbon 47 from second securing tape ribbon 67 when removing the diaper from the infant. Distal end portion 117 of free working end 50 of first securing tape ribbon 47 projects beyond the outermost edge 118 of adhesive coating 61 on free working end 50. Projecting portion 117 provides a gripping means for separating the adhesive- coated free end 50 of first securing tape ribbon 47 from second securing tape ribbon 67.

To facilitate separation of first and second tape ribbons 47 and 67, outer face 69 of second securing tape ribbon 67 preferably is slightly treated with a release compound to permit a relatively strong adhesive attachment between the tape ribbons while simultaneously enabling free working end 50 of first securing tape ribbon 47 to be separated from second securing tape ribbon 67. Alternatively, second securing tape ribbon 67 may be a material having the desired degree of inherent release properties. If desired, adhesive coatings 61 and 62 on first securing tape ribbon 47 may be pressure-sensitive and together comprise a substantially continuous adhesive coating on inner face 48 of first securing tape ribbon 47. Adhesive tabs suitable for the purposes of the present invention can be made from a wide variety of materials, provided that such materials are sufficiently flexible. Preferred materials for this purpose are polyalkylene webs such as polyethylene sheet, polypropylene sheet, and the like. Particularly preferred are webs which are oriented along the narrow dimension of the tab or webs which have filament reinforcements therein.

The pressure-sensitive layers such as adhesive coatings 61 and 73 are provided by applying a coating of a pressure-sensitive adhesive composition known in the art to be the appropriate surfaces of tape ribbons 47 and 67. The applied adhesive shall have good tack, good cohesive strength, good resistance to moisture and good resistance to aging. Illustrative of such adhesive compositions are mixtures of natural or synthetic rubber, zinc oxide, and various resins, also lattices of natural or synthetic rubber, or water dispersions of acrylic tacky polymers or copolymers, and the like.

Anchored release strips can be made from smooth plastic film having a relatively non-adhering surface, from paper coated with a silicone release compound, or from similar release materials. A number of appropriate release coatings may be used with the present invention.

Figure 4 is an illustration of another fastener embodiment of the present invention. The embodiment of Figure 4 is identical to the above embodiments except for the differences mentioned below. In the embodiment of Figure 4 fastener 40, shown in the working position, includes a first securing tape ribbon 47 having an inner face 48 and an outer face 49. First securing tape ribbon 47 has a free working end 50 and a fixed end 52 which is permanently attached to a marginal portion of diaper 20, preferably along backsheet 26.

Fixed end 52 is provided with adhesive coating 62 on inner face 48 thereof. Fixed end 52 of first securing tape ribbon 47 is attached to backsheet 26 by means of adhesive coating 62 which can be made of a pressure-sensitive adhesive composition, a heat-activated or solvent-activated adhesive composition, or the like.

Securement means 60 is provided on one face of free working end 50. As shown in Figure 4, securement means 60 on free working end 50 comprises a plurality of hooks 280. Hooks 280 are coextensive with free working end 50 but for a grippable distal end portion 117.

Tab 40 further includes second securing tape ribbon 67. Second securing tape ribbon 67 comprises a loop material 270 engageable with the hooks 280. The attachment or bond between second tape ribbon 67 and free working end 50 is at least as strong as the attachment or bond between fixed end 52 and backsheet 26 so that ribbons 47 and 67 effectively perform as a single tab or ribbon during the initial diaper securement. Grippable distal end portion 117 of first securing tape ribbon 47 extends beyond first end edge 70 of second securing tape ribbon 67. A continuous pressure-sensitive adhesive coating 73 is provided along a portion of inner face 68 of second securing tape ribbon 67.

The second securing tape ribbon 67 projects beyond an end edge of said pressure-sensitive adhesive coating 73 to provide a finger lift 300. Finger lift 300 is positioned at the end remote from grippable distal end portion 117.

The diaper 20 may also comprise side panels 30. The side panels 30 may be elastic or extensible to provide a more comfortable and contouring fit by initially conformably fitting the diaper 20 to the wearer and sustaining this fit throughout the time of wear well past when the diaper 20 has been loaded with exudates since the elasticized side panels 30 allow the sides of the diaper 20 to expand and contract. The side panels 30 may also provide more effective application of the diaper 20 because even if the diaperer pulls one elasticized side panel 30 farther than the other during application, the diaper 20 will "self-adjust" during wear.

While the diaper 20 of the present invention preferably has the side panels 30 disposed in the second waist region 38, the diaper 20 may be provided with side panels 30 disposed in the first waist region 36 or in both the first waist region 36 and the second waist region 38. The side panels 30 may be constructed in any suitable configurations. Examples of diapers with elasticized side panels are disclosed in U.S. Patent 4,857,067, entitled "Disposable Diaper Having Shirred Ears" issued to Wood, et al. on August 15, 1989; U.S. Patent 4,381,781 issued to Sciaraffa, et al. on May 3, 1983; U.S. Patent 4,938,753 issued to Van Gompel, et al. on July 3, 1990; the herein before referenced U.S. Pat. No. 5,151,092 issued to Buell on September 9, 1992; and U.S. Pat. No. 5, 221,274 issued to Buell on June 22, 1993; U.S. Patent No. 5,669,897 issued to LaVon, et al. on September 23, 1997 entitled "Absorbent Articles Providing Sustained Dynamic Fit"; U.S. Patent Application Serial No. 08/155,048 entitled "Absorbent Article With Multi-Directional Extensible Side Panels" filed November 19, 1993 in the names of Robles, et al.; each of which is incorporated herein by reference.

The diaper 20 preferably further includes leg cuffs 32 which provide improved containment of liquids and other body exudates. Leg cuffs may also be referred to as leg bands, side flaps, baffler cuffs, or elastic cuffs. U.S. Patent 3,860,003 describes a disposable diaper which provides a contractible leg opening having a side flap and one or more elastic members to provide an elasticized leg cuff (a gasketing cuff). U.S. Patent Nos. 4,808,178 and 4,909,803 issued to Aziz et al. on February 28, 1989 and March 20, 1990, respectively, describe disposable diapers having "stand-up" elasticized flaps (barrier cuffs) which improve the containment of the leg regions. U.S. Patents 4,695,278 and 4,795,454 issued to Lawson on September 22, 1987 and to Dragoo on January 3, 1989, respectively, describe disposable diapers having dual cuffs, including gasketing cuffs and barrier cuffs.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1. A fastener (40) which comprises:
a first securing tape ribbon (47) having a free working end (50), a securement means (60) on at least one face (48) of said free working end (50);
a second securing tape ribbon (67) being coextensive with and releasably attached to said securement means (60) of said first securing tape ribbon (47), and a pressure-sensitive adhesive coating (73) on said second securing ribbon (67) on the face thereof opposite to the face attached to said free working end (50) of said first securing tape ribbon (47); and
release means (74);
said second securing tape ribbon (67) being movable from a storage position in which said second securing tape ribbon (67) is releasably adhered to said release means (74) to a working position;
said free working end (50) of said first securing tape ribbon (47) being separable from said second securing tape ribbon (67) to make said securement means (60) on said free working end (50) of said first securing tape ribbon (47) available for use in refastening; and
characterized in that a grippable portion (117) of said free working end (50) projects beyond the respective outermost edges of said release means (74) and said second securing tape ribbon (67).

2. The fastener of claim 1 wherein said second securing tape ribbon (67) comprises a loop material (270).

3. The fastener of claim 2 wherein said securement means (60) comprises a plurality of hooks (280) engageable with said loop material.

4. The fastener of claim 1 wherein said second securing tape ribbon (67) projects beyond an end edge of said pressure-sensitive adhesive coating (73) to provide a finger lift (300).

5. A disposable absorbent article (20) comprising a topsheet (24), a moisture-impervious backsheet (26) substantially coextensive with said topsheet, an absorbent core (28) positioned between said topsheet and said backsheet, and an adhesive tab fastener (40) which comprises:
a first securing tape ribbon (47) having a fixed end (52) and a free working end (50);
an adhesive coating (62) on at least one face (48) of said fixed end (52) of said first securing tape ribbon by means of which said fixed end of said first securing tape ribbon is attached to said disposable absorbent article, and a pressure-sensitive adhesive coating (61) on said one face of said free working end (50) of said first securing tape ribbon;
a second securing tape ribbon (67) being coextensive with and adhesively but releasably attached to said adhesive-coated face of said free working end (50) of said first securing tape ribbon (47), and a pressure-sensitive adhesive coating (73) on said second securing ribbon (67) on the face thereof opposite to the face attached to said free working end (50) of said first securing tape ribbon (47); and
release means (74) on said topsheet (24);
said second securing tape ribbon (67) being movable from a folded-over storage position in which said second securing tape ribbon (67) is releasably adhered to said release means (74) to a working position in which said adhesive-coated second securing tape ribbon is available for use in securing said disposable absorbent article about an infant;
said free working end (50) of said first securing tape ribbon (47) being separable from said second securing tape ribbon (67) to enable said disposable absorbent article to be removed from an infant and to make said pressure-sensitive adhesive coating (61) on said free working end (50) of said first securing tape ribbon (47) available for use in refastening said disposable absorbent article about an infant; and
characterized in that a grippable portion (117) of said free working end (50) projects beyond the respective outermost edges of said release means (74) and said second securing tape ribbon (67).

6. The fastener of claim 5 wherein said second securing tape ribbon (67) projects beyond an end edge of said pressure-sensitive adhesive coating (73) to provide a finger lift (300).
